# EUROPEAN PATENT SPECIFICATION

(11) **EP 1 387 689 B1**
(45) Date of publication and mention of the grant of the patent: **02.11.2006**
(21) Application number: 02740542.2
(22) Date of filing: 30.04.2002
(51) Int. Cl.: A61K 33/24, A61K 31/70, A61K 31/515, A61K 45/06, A61P 35/04, A61P 35/00

(54) **COMBINATION OF A GELATINASE INHIBITOR AND AN ANTI-TUMOR AGENT, AND USES THEREOF**
KOMBINATION EINES GELATINASEHEMMERS UND EINES ANTI-TUMOR-AGENT, UND DEREN VERWENDUNG
COMBINAISON D'UN INHIBITEUR DE GELATINASE ET D'UN ANTITUMORAL, ET SES UTILISATIONS

(30) Priority: 03.05.2001 EP 01110119
(43) Date of publication of application: 11.02.2004
(73) Proprietor: F. HOFFMANN-LA ROCHE AG, 4070 Basel (CH)
(72) Inventor: FRIESS, Thomas, 82152 Planegg (DE); KRELL, Hans-Willi, 82377 Penzberg (DE); SCHEUER, Werner, 82393 Iffeldorf (DE); TIEFENTHALER, Georg, 82404 Sindelsdorf (DE)
(74) Representative: Schreiner, Siegfried
(86) International application number: PCT/EP2002/004744
(87) International publication number: WO 2002/089824

(56) References cited:
- WO-A-00/37107
- WO-A-97/23465
- WO-A-98/58925
- WO-A-99/52889
- SHALINSKY, DAVID R. ET AL: "Marked antiangiogenic and antitumor efficacy of AG3340 in chemoresistant human non-small-cell lung cancer tumors: single agent and combination chemotherapy studies" CLIN. CANCER RES. (1999), 5(7), 1905-1917, XP001042322
- YIP, DESMOND ET AL: "Matrix metalloproteinase inhibitors: applications in oncology" INVEST. NEW DRUGS (1999), 17(4), 387-399, XP001058160
- SHALINSKY D R ET AL: "BROAD ANTITUMOR AND ANTIANGIOGENIC ACTIVITIES OF AG3340, A POTENT AND SELECTIVE MMP INHIBITOR UNDERGOING ADVANCED ONCOLOGY CLINICAL TRIALS" ANNALS OF THE NEW YORK ACADEMY OF SCIENCES, NEW YORK ACADEMY OF SCIENCES, NEW YORK, NY, US, vol. 878, 1999, pages 236-270, XP001007565 ISSN: 0077-8923
- GIAVAZZI ET AL: "Batimastat, a synthetic inhibitor of matrix metalloproteinases, potentiates the antitumor activity of cisplatin in ovarian carcinoma xenaografts" CLIN. CANCER RES., vol. 4, 1998, pages 985-992, XP001042280 cited in the application
- HAQ, MAHMUDUL ET AL: "Addition of matrix metalloproteinase inhibition to conventional cytotoxic therapy reduces tumor implantation and prolongs survival in a murine model of human pancreatic cancer" CANCER RES. (2000), 60(12), 3207-3211, XP001042323
- BROWN P D ET AL: "MATRIX METALLOPROTEINASE INHIBITION: A REVIEW OF ANTI-TUMOUR ACTIVITY" ANNALS OF ONCOLOGY, KLUWER, DORDRECHT, NL, vol. 6, 1995, pages 967-974, XP000992860 ISSN: 0923-7534
- TAMURA Y ET AL: "HIGHLY SELECTIVE AND ORALLY ACTIVE INHIBITORS OF TYPE IV COLLAGENASE (MMP-9 AND MMP-2): N-SULFONYLAMINO ACID DERIVATIVES" JOURNAL OF MEDICINAL CHEMISTRY, AMERICAN CHEMICAL SOCIETY. WASHINGTON, US, vol. 41, no. 4, 1998, pages 640-649, XP002072052 ISSN: 0022-2623
- BROWN P D: "CLINICAL STUDIES WITH MATRIX METALLOPROTEINASE INHIBITORS" APMIS, COPENHAGEN, DK, vol. 107, 1999, pages 174-180, XP001008373 ISSN: 0903-4641
- ROYER, ISABELLE ET AL: "Metabolism of docetaxel by human cytochromes P450: interactions with paclitaxel and other antineoplastic drugs" CANCER RES. (1996), 56(1), 58-65, XP001042324
- KELLY, KAREN: "New chemotherapy agents for small cell lung cancer" CHEST (2000), 117(4, SUPPL. 1), 156S-162S, XP001064989

## Description

The present invention relates to composition and treatments of patients with solid metastasized or non-metastasized tumors. These are characterized by administration of a gelatinase inhibitor, RO 28-2653, in combination with a cytotoxic/cytostatic compound, e.g. Cisplatin, Paclitaxel, Gemcitabine or Etoposide.

### Introduction

In modern clinical oncology, the biggest challenge for the successful treatment of patients is the problem of metastasis rather than the primary tumor itself. For tumor cells to be able to spread and form distant metastases several prerequisites have to be fulfilled. Among these one of the most important ones is the ability to grow invasively into the surrounding tissue, intravasate into the blood- or lymphatic vessel system and finally to extravasate and seed in the target tissue.

Recently a new class of molecules, namely the proteases, were identified to play a major role in this process. With the help of these enzymes tumor cells break down extracellular matrix proteins which are major constituents of connective tissue and basal membranes. Among these proteases the matrix metalloproteases (MMPs), and, more specifically, MMP-2 and MMP-9 (= gelatinases A and B) were identified as major contributors in this process of matrix degradation (Johansson et al., Cell. Mol. Life Sci. 57 (2000) 5-15). In addition, especially MMP-9 was found to play an important role in the formation of new blood vessels, a process called angiogenesis, which is essential for a tumor to establish and uphold a sufficient supply with nutrients and oxygen (Vu, T.H., et al., Cell 93 (1998) 411-422). Not surprisingly, indeed MMP-2 and/or MMP-9 were found to be overexpressed by a large proportion of individual tumors irrespective of histological origin.

Inhibition of MMPs, either with the naturally occurring Tissue Inhibitors of Metalloproteases (TIMPs), or with low molecular weight inhibitors, resulted in impressive anti-tumor and anti-metastatic effects in animal models (Brown, P.D., Medical Oncology 14 (1997) 1-10). Most of the low-molecular weight inhibitors of MMPs are derived from the hydroxamic acid compound class and inhibit MMPs in a broad manner, being not selective for MMP-2 and MMP-9, the key MMPs in tumor invasion, metastatic spread, and angiogenesis. However, MMP inhibiting molecules from various other structural classes, e.g. the tri-oxo pyrimidines, have been described, e.g. in WO 97/23465 and WO 01/25217. A member of this class of compounds, RO 28-2653, is an extremely potent, and highly selective, gelatinase inhibitor with an almost exclusive specificity for MMP-2, MMP-9, and MT1-MMP, the enzyme activating MMP-2, while sparing most other members of the MMP family of proteases. Ro 28-2653, with the chemical name 5-(4-biphenyl)-5-[N-(4-nitrophenyl) piperazinyl] barbituric acid is described in WO 97/23465.

Several MMP inhibitors, predominantly of the hydroxamic acid substance class were, and in part still are, in clinical testing. All of the published clinical results with these inhibitors were disappointing, showing little or no clinical efficacy (Fletcher, L., Nature Biotechnology 18 (2000) 1138-1139). The reason for this lack of efficacy in the clinic most likely is the fact that patients could not be given high enough doses for anti-tumor or anti-metastatic activity because of the side effects associated with these broadly acting inhibitors. These dose-limiting side effects were predominantly arthralgias and myalgias (Drummond, A.H., et al., Ann. N.Y. Acad. Sci. 878 (1999) 228-235). As a possible way to circumvent this problem, the combination of MMP inhibitors with classical cytostatic/cytotoxic compounds was evaluated in animal studies. Indeed, in these experiments, MMP inhibitors, in combination with cytostatic/cytotoxic drugs, showed enhanced efficacy (Giavazzi, R., et al., Clin. Cancer Res. 4 (1998) 985-992).

Ro 28-2653 is an MMP inhibitor with high selectivity for MMP-2 and MMP-9 and the treatment with this compound showed no side effects. Indeed, no side effects similar to those observed with the broad-spectrum inhibitors were seen in toxicological tests over a wide range of doses. Thus, no dose-limiting toxicities were expected with RO 28-2653, and accordingly no additional benefit from co-treatment with cytostatic/cytotoxic drugs was expected. However, to explore also the distant possibility of an additional benefit from combination treatment, such studies were initiated and conducted in various animal models. The models and cytostatic/cytotoxic drugs were chosen to reflect as broad a spectrum of oncological indications and clinically active treatment principles as possible.

Surprisingly, combinations of RO 28-2653 with cytostatic/cytotoxic compounds in various models of different histological origin clearly showed enhanced anti-tumor activity as compared to the respective single-agent treatments. Thus, in principle all human patients with solid metastasized or non-metastasized tumors, e.g. tumors of the lung, prostate, colon, breast, pancreas, ovary, skin, kidney, bladder, liver, head and neck, stomach, and brain are eligible for treatment with gelatinase inhibitors in combination with cytotoxic/cytostatic compounds.

The treatment with the gelatinase-inhibitor most likely will be a chronic treatment, starting either simultaneously with the combination partner or sequentially, i.e. before and after the treatment with the combination partner. In this context, simultaneous treatment means that the gelatinase inhibitor treatment takes place in parallel to, and is not stopped for, the necessary cycles of cytostatic/cytotoxic treatment, while sequential treatment means that the gelatinase inhibitor treatment is discontinued for the duration of the treatment with the cytostatic/cytotoxic drugs. The administration schedule depends on the tumor to be treated as well as on the cytostatic/cytotoxic agent to be used.

Preferred cytostatic/cytotoxic compounds are, for example: Cisplatin, Paclitaxel, Vinblastin, Mitomycin, Gemcitabine, Etoposide, Doxetaxel, Carboplatin, Irinotecan, Topotecan, Navelbine, Doxorubicin, Epirubicin, Oxaliplatin, 5-Fluoruracil, Capecitabine, 5-UFT, Herceptin, alpha interferon.

The administration of the gelatinase inhibitor will preferentially be oral, with doses ranging between 0.5 mg/kg and 50 mg/kg. Administration of the various combination partners will be as approved by the health authorities, which in most cases is by i.v. infusion. The partners used for the combination therapy can be contained in separate package format or together in a kit. Such a kit contains the i.v. preparations of the cytotoxic/cytostatic agents, e.g. ampoules and blister packages with tablets of the gelatinase inhibitors.

The following experimental part, references and figures are provided to aid the understanding of the present invention, the true scope of which is set forth in the appended claims. It is understood that modifications can be made in the procedures set forth without departing from the spirit of the invention.

### Description of the Figures

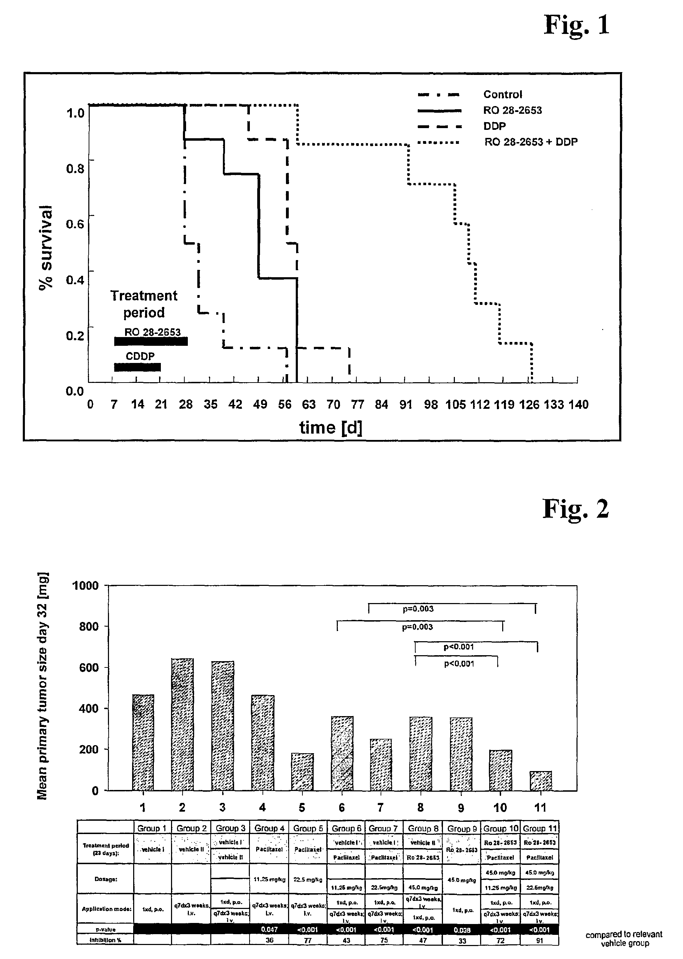

- Figure 1: shows the effect of the combination of RO 28-2653 and Cisplatin on survival in the orthotopic HOC-22 ovarian cancer xenograft model. DDP = Cisplatin. Survival is displayed as Kaplan-Meyer-Plot. Statistics was calculated using the log rank test. Animals were treated with RO 28-2653 for three weeks, from day 7 to day 21, with daily oral doses of 45 mg/kg six times a week. Cisplatin treatment consisted of 4 doses of 3 mg/kg i.v. per mouse once every 4 days, starting on day 7.

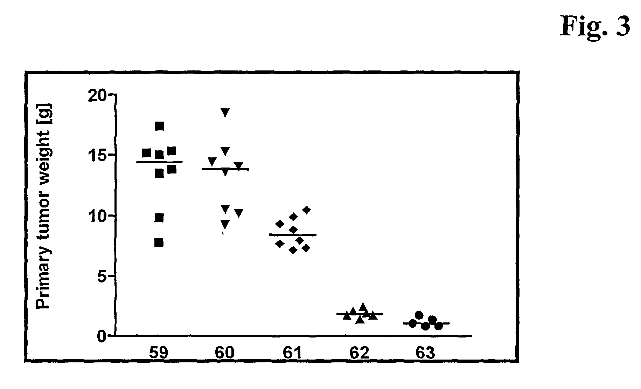

- Figure 2: shows the effect of the combination of RO 28-2653 and Paclitaxel on primary tumor size in the subcutaneous HCT116 CL5.5 colon cancer xenograft model.
- Figure 3: shows the effect of the combination of RO 28-2653 and Etoposide on the weight of primary tumors in the syngeneic orthotopic rat MatLyLu prostate cancer model. ■ Untreated Control ▼ Vehicle Control ◆ Etoposide ▲. RO 28-2653 ● RO 28-2653 + Etoposide. ― Mean tumor weight. Rats were treated with RO 28-2653 with daily oral doses of 100 mg/kg starting on day 6 after tumor implantation, until the penultimate day of the experiment (day 17). Etoposide was given intraperitoneally once daily, from day 6 to day 17, at a dose of 25 mg/m².

### Experimental Part

### Combination of RO 28-2653 with Cisplatin

The activity of RO 28-2653 in combination with Cisplatin was evaluated in the human orthotopic ovarian carcinoma mouse xenograft model HOC-22. While control mice had a median survival time of 30 days, treatment with RO 28-2653 for three weeks, or Cisplatin for two weeks as single agents, resulted in an increase in lifespan of 63% and 95%, respectively. When used in combination, however, an increase in lifespan of 263% versus vehicle and 86% versus Cisplatin alone was observed (Fig. 1). Thus, RO 28-2653, when given in combination with Cisplatin, was able to potentiate its anti-tumor effect significantly and increase the survival time of the animals.

### Combination of RO 28-2653 with Paclitaxel

The activity of RO 28-2653 in combination with Paclitaxel was evaluated in the human subcutaneous colon carcinoma mouse xenograft model HCT 116 Cl 5.5 with primary tumor size as the endpoint. Animals from the Paclitaxel monotherapy group had an inhibition of primary tumor growth by 43% and 75% at the doses of 11.5 and 22.5 mg/kg, respectively. RO 28-2653 monotherapy resulted in the inhibition of primary tumor growth by 74% at the dose of 45 mg/kg. Combination therapy with both Paclitaxel and RO 28-2653 significantly inhibited primary tumor growth by 72% and 91% for the Paclitaxel doses of 11.5 and 22.5 mg/kg, respectively, with a dose of 45 g/kg for RO 28-2653 (Fig. 2). Thus, the combination treatment of RO 28-2653 with Paclitaxel resulted in a significant benefit for the experimental animals with respect to primary tumor size.

### Combination of RO 28-2653 with Gemcitabine

The activity of RO 28-2653 in combination with Gemcitabine was evaluated in the human orthotopic pancreas carcinoma mouse xenograft model PancTul with primary tumor size and number and size of metastases as endpoints. Animals from the Gemcitabine monotherapy group had an inhibition of primary tumor growth by 85%. RO 28-6253 monotherapy resulted in the inhibition of primary tumor growth by 66%. Importantly, combination therapy with both Gemcitabine and RO 28-2653 significantly inhibited primary tumor growth by 94% (Table 1). With respect to the number of metastases, in the untreated or vehicle treated control groups an average of 5.1 and 4.6 metastases per animal was found. While RO 28-2653 treatment reduced these numbers to an average of 2.5 metastases per animal, and Gemcitabine monotherapy to 0.4 metastases, combination treatment with Gemcitabine plus RO 28-2653 reduced this number even further to 0.08 metastases per animal (one single metastasis in the entire treatment group) (Table 2). This is a further 5-fold reduction of the number of metastases beginning at an already low level, which makes this reduction even more impressive. This antimetastatic effect could be, at least in part, due to anti-angiogenic effects exerted by the gelatinase-inhibitor. In fact, a defect in neo-angiogenesis has been described for gelatinase B defective mice, thus corroborating this hypothesis.

**Table 1**

| | | **No treatment** | **Vehicle 1 + 2** | **Gemcitabine + Vehicle 2** | **Ro28-2653 + Vehicle 1** | **Gemcitabine + Ro28-2653** |
|---|---|---|---|---|---|---|
| | | n=9 | n=10 | n=13 | n=13 | n=13 |
| **Tumor take** | Primary | 9/9 | 10/10 | 13/13 | 13/13 | 13/13 |
| **rate** | tumor | | | | | |
| **Volume** | Primary | **Vm=293(±79)** | **Vm=333(±87)** | **Vm=51(±14)** | **Vm=112(±46)** | **Vm=20(±4)** |
| | tumor | **mm**^{**3**} | **mm**^{**3**} | **mm**^{**3**} | **mm**^{**3**} | **mm**^{**3**} |
| **Necrosis** | Primary | 0/9 | 0/10 | 4/13 | 1/13 | 9/13 |
| | tumor | | | | | |
| **Body weight** | | m=-10 (±5)% | m=-11 (±7)% | m=-2 (±5)% | m=-5 (±4)% | m=-1 (±4)% |

Effect of the combination of RO 28-2653 and Gemcitabine on primary tumor volume in the orthotopic PancTul pancreas cancer xenograft model. Mice were treated with RO 28-2653 with daily oral doses of 45 mg/kg from day 7 until day 30. Gemcitabine treatment consisted of one intraperitoneal dose of 2.2 mg/kg every second day from day 7 to day 30.

**Table 2**

| | **No treatment** | **Vehicle 1 + 2** | **Gemcitabine + Vehicle 2** | **Ro28-2653 + Vehicle 1** | **Gemcitabine + Ro28-2653** |
|---|---|---|---|---|---|
| | n=9 | n=10 | n=13 | n=13 | n=13 |
| **Metastasis** | | | | | |
| Lung/ Mediastinum | 7/9 | 4/10 | 0/13 | 2/13 | 0/13 |
| | | | | | |
| Liver, in parenchyme | 3/9 | 3/10 | 1/13 | 3/13 | 0/13 |
| Liver, on serosa | 1/9 | 1/10 | 1/13 | 2/13 | 0/13 |
| Liver hilus | 1/9 | 2/13 | 0/13 | 4/13 | 0/13 |
| Kidneys/Adrenal gland (capsule) | 4/9 | 3/10 | 0/13 | 1/13 | 0/13 |
| Spleen (serosa), gastrosplenic ligament | 3/9 | 3/10 | 0/13 | 0/13 | 0/13 |
| Lymph nodes in mesentery | 2/9 | 2/10 | 0/13 | 2/13 | 0/13 |
| Mesentery < 3 metastasis(2 mm³) | 1/9 | 0/10 | 0/13 | 0/13 | 0/13 |
| Mesentery 3-20 metastasis(1-18 mm³) | 7/9 | 7/10 | 0/13 | 5/13 | 0/13 |
| Ligament of the uterus / testis (serosa), seminal vesicles | 5/9 | 3/10 | 0/13 | 0/13 | 0/13 |
| Diaphragm | 3/9 | 5/10 | 0/13 | 3/13 | 0/13 |
| Pelvis | 1/9 | 3/10 | 0/13 | 0/13 | 0/13 |
| Site of surgical incision | | | | | |
| Small < 10 mm³ | 0/9 | 1/10 | 2/13 | 2/13 | 1/13 |
| Medium < 50 mm³ | 0/9 | 1/10 | 1/13 | 3/13 | 0/13 |
| Large 80-280 mm³ | 8/9 | 8/10 | 0/13 | 6/13 | 0/13 |

Effect of the combination of RO 28-2653 and Gemcitabine on metastatic spread in the orthotopic PancTu1 pancreas cancer xenograft model. Mice were treated with RO 28-2653 with daily oral doses of 45 mg/kg from day 7 until day 30. Gemcitabine treatment consisted of one intraperitoneal dose of 2.2 mg/kg every second day from day 7 to day 30.

### Combination of RO 28-2653 with Etoposide

The activity of RO 28-2653 in combination with Etoposide was evaluated in the rat syngeneic orthotopic prostate carcinoma model MatLyLu with primary tumor size as endpoint. Animals from the Etoposide monotherapy group showed inhibition of primary tumor growth by 35% as compared to the vehicle-treated animals. RO 28-6253 monotherapy resulted in the inhibition of primary tumor growth by 86%. Importantly, the combination therapy with both Etoposide and RO 28-2653 significantly inhibited primary tumor growth by 92% (Fig. 3).

### List of References

Brown, P.D., Medical Oncology 14 (1997) 1-10
Drummond, A.H., et al., Ann. N.Y. Acad. Sci. 878 (1999) 228-235
Fletcher, L., Nature Biotechnology 18 (2000) 1138-1139
Giavazzi, R., et al., Clin. Cancer Res. 4 (1998) 985-992
Johansson, N., et al., Cell. Mol. Life Sci. 57 (2000) 5-15
Vu, T.H., et al., Cell 93 (1998) 411-422
WO 01/25217
WO 97/23465

## Claims

1. Use of the gelatinase inhibitor 5-(4-biphenyl)-5-[N-(4-nitrophenyl) piperazinyl] barbituric acid in combination with an antitumor agent for the preparation of a medicament for the treatment of tumor growth or inhibiting metastases.

2. Use according to claim 1, wherein the antitumor agent is a compound selected from the group consisting of Cisplatin, Paclitaxel, Vinblastin, Mitomycin, Gemcitabine, Etoposide, Doxetaxel, Carboplatin, Irinotecan, Topotecan, Navelbine, Doxorubicin, Epirubicin, Oxaliplatin, 5-Fluoruracil, Capecitabine, 5-UFT, Herceptin, alpha interferon.

3. Use according to claims 1 to 2, whereby the gelatinase inhibitor and the tumor agent are to be administered simultaneously.

4. Use according to claims 1 to 2, whereby the gelatinase inhibitor and the tumor agent are to be administered sequentially.

5. Use according to claims 1 to 3, whereby the gelatinase inhibitor and the anti-tumor agent are part of a kit.

6. Use according to claims 1 to 5, wherein the gelatinase inhibitor is present as a tablet or capsule.

## Patentansprüche

1. Verwendung des Gelatinaseinhibitors 5-(4-biphenyl)-5-[N-(4-nitrophenyl) piperazinyl] Barbitursäure in Kombination mit einem Antitumor-Agenz zur Herstellung eines Medikaments für die Behandlung von Tumorwachstum oder das Inhibieren von Metastasen.

2. Verwendung gemäß Anspruch 1, worin das Antitumor-Agenz eine Verbindung, ausgewählt aus der Gruppe, bestehend aus Cisplatin, Paclitaxel, Vinblastin, Mitomycin, Gemcitabin, Etoposid, Doxetaxel, Carboplatin, Irinotecan, Topotecan, Navelbin, Doxorubicin, Epirubicin, Oxaliplatin, 5-Fluoruracil, Capecitabin, 5-UFT, Herceptin, Alpha-Interferon ist.

3. Verwendung gemäß den Ansprüchen 1 bis 2, worin der Gelatinaseinhibitor und das Antitumor-Agenz simultan zu verabreichen sind.

4. Verwendung gemäß den Ansprüchen 1 bis 2, worin der Gelatinaseinhibitor und das Antitumor-Agenz aufeinanderfolgend zu verabreichen sind.

5. Verwendung gemäß den Ansprüchen 1 bis 3, worin der Gelatinaseinhibitor und das Antitumor-Agenz Teil eines Kits sind.

6. Verwendung gemäß den Ansprüchen 1 bis 5, worin der Gelatinaseinhibitor als eine Tablette oder Kapsel vorliegt.

## Revendications

1. Utilisation de l'acide 5-(4-biphényl)-5-[N-(4-nitrophényl) pipérazinyle] barbiturique, inhibiteur de la gélatinase, en combinaison avec un agent antitumoral pour la préparation d'un médicament destiné au traitement de la croissance tumorale ou pour inhiber les métastases.

2. Utilisation selon la revendication 1, dans laquelle l'agent antitumoral est un composé choisi dans le groupe consistant en Cisplatine, Paclitaxel, Vinblastine, Mitomycine, Gemcitabine, Etoposide, Doxetaxel, Carboplatine, Irinotecan, Topotecan, Navelbine, Doxorubicine, Epirubicine, Oxaliplatine, 5-Fluoruracile, Capécitabine, 5-UFT, Herceptine, alpha-interféron.

3. Utilisation selon les revendications 1 à 2, dans laquelle l'inhibiteur de la gélatinase et l'agent tumoral doivent être administrés simultanément.

4. Utilisation selon les revendications 1 à 2, dans laquelle l'inhibiteur de la gélatinase et l'agent tumoral doivent être administrés séquentiellement.

5. Utilisation selon les revendications 1 à 3, dans laquelle l'inhibiteur de la gélatinase et l'agent antitumoral font partie d'un kit.

6. Utilisation selon les revendications 1 à 5, dans laquelle l'inhibiteur de la gélatinase est présent sous la forme de comprimé ou de gélule.
